# EUROPEAN PATENT APPLICATION

(11) **EP 2 668 971 A1**
(43) Date of publication of application: **04.12.2013**
(21) Application number: 12187807.8
(22) Date of filing: 09.10.2012
(51) Int. Cl.: A61M 16/06

(54) **Adjustable respiratory mask**

(30) Priority: 31.05.2012 TW 101210437; 03.07.2012 TW 101212856
(71) Applicant: Galemed Corporation, 114 Taipei (TW)
(72) Inventor: Lee, Gary C. J., 114, Taipei (TW)
(74) Representative: 2K Patentanwälte Blasberg Kewitz & Reichel

(57) **Abstract**

An adjustable respiratory mask includes a nasal cover (1) and a forehead fixing base (2). The nasal cover (1) includes a frame assembly (11) and a cover body (12). The frame assembly (11) has an upright frame (111) and an adjustable support (112) extending from the upright frame (111). The upright frame (111) is connected to the cover body (12). The adjustable support (112) is a plastic piece and inclined relative to the upright frame (111). The forehead fixing base (2) has a base body (21) provided with a receiving portion (213). The adjustable support (112) penetrates the receiving portion (213) to allow the base body (21) to adjustably move. Thus, the respiratory mask of the present invention can fit face contours of different users, so that the users can feel more stable and comfortable when wearing it.

## Description

### BACKGROUND OF THE INVENTION

### FIELD OF THE INVENTION

The present invention relates to a respiratory mask, and in particular to an adjustable respiratory mask.

### DESCRIPTION OF PRIOR ART

Obstructive Sleep Apnea (OSA) is a sleep disorder caused by obstruction of the upper airway. It is characterized by repetitive pauses in breathing during sleep, and is usually associated with a reduction in blood oxygen condensation. Due to multiple times of sleep disturbance and low blood oxygen condensation, those who suffer from the OSA has greater possibility of suffering from cardiovascular diseases than normal people.

The most effective way of curing the OSA is to use a continuous positive airway pressure (CPAP) respirator, which comprises a mask and an air supply device connected to the mask. The mask includes a nasal cover, a forehead fixing base, and a supporting frame connecting the nasal cover and the forehead fixing base. By this arrangement, the air supply device continuously pumps the air into the nasal cover to generate a positive-pressure continuous airflow to the user. In this way, the upper airway of the patent can be kept unobstructed and the sleep apnea can be cured.

However, since the supporting frame between the nasal cover and the forehead fixing base is not adjustable, the supporting frame cannot be adjusted to correspond to the face contours of different users. As a result, the forehead fixing base cannot fit the forehead of the user. Further, the user who wearing such a mask will feel uncomfortable. Even, the oxygen gas may leak from the nasal cover, so that air pressure is insufficient.

In view of the above, the present Inventor proposes a novel and reasonable structure based on his expert knowledge and deliberate researches.

The present invention is to provide an adjustable respiratory mask, which has an adjustable support to adjust the distance between the nasal cover and the forehead fixing base. The adjustable support is a plastic piece and inclined relative to an upright frame, thereby adjusting the angle between the nasal cover and the forehead fixing base. In this way, the face contours of different users can be fitted, and the user wearing such a respiratory mask can feel more stable and comfortable.

The present invention is to provide an adjustable respiratory mask, including: a nasal cover comprising a frame assembly and a cover body, the frame assembly having an upright frame and an adjustable support extending from the upright frame, the upright frame being connected to the cover body, the adjustable support being a plastic piece and inclined relative to the upright frame; and a forehead fixing base having a base body, the base body being provided with a receiving portion, the adjustable support penetrating the receiving portion to allow the base body to adjustably move.

The present invention has the following advantageous features:

The adjustable support movably penetrates the receiving portion to thereby adjust the distance between the nasal cover and the forehead fixing base. The adjustable support is a plastic piece and inclined relative to the upright frame, thereby adjusting the angle between the nasal cover and the forehead fixing base. Thus, the respiratory mask of the present invention can fit the face contours of different users, and make the users to feel stable and comfortable when wearing it.

Further, the adjustable support is a plastic piece, so that the adjustable support can swing relative to the upright frame when the adjustable support is used. In this way, the angle between the nasal cover and the forehead fixing base can be adjusted to fit the face contours of different users. However, the plastic piece may suffer from an elastic fatigue after being used for a period of time, thereby memorizing the special face contour of the user, thereby making the user to feel more stable and comfortable when wearing it.

An engaging piece is engaged with any one of engaging troughs, thereby adjusting the distance between the nasal cover and the forehead fixing base via the adjusting support. Further, the angle between the nasal cover and the forehead fixing base can be adjusted, so that the forehead fixing base can be slightly adjusted in height besides its adjustment in the front-and-rear direction. In this way, the nasal cover and the forehead fixing base will more fit the face contour of the user, so that the cover portion can tightly cover the nose and mouth of the user to avoid the insufficient air pressure due to air leakage. Thus, the respiratory mask of the present invention has an increased stability in use.

Further, the respiratory mask of the present invention further includes a protective pad made of silicon or rubber. The protective pad is elastically sandwiched between the forehead fixing base and the forehead of the user. No matter the user wears the respiratory mask tight or loose, the protective pad can still provide a cushioning effect to the user, so that the user can feel more comfortable when wearing it.

The respiratory mask of the present invention further includes a connecting pipe and a head band. The head band is provided with a penetrating portion to form a space between the head band and the head of the user. By this arrangement, the connecting pipe can penetrate the penetrating portion to be fixed thereto easily without shaking. Thus, the respiratory mask of present invention can be used more conveniently.

### BRIEF DESCRIPTION OF DRAWING

FIG. 1 is an exploded perspective view showing the respiratory mask of the present invention;
FIG. 2 is an assembled view showing the respiratory mask of the present invention;
FIG. 3 is another assembled view showing the respiratory mask of the present invention;
FIG. 4 is a schematic view showing an operating state of the respiratory mask of the present invention;
FIG. 5 is a schematic view showing another operating state of the respiratory mask of the present invention; and
FIG. 6 is a schematic view showing an operating state of the respiratory mask in accordance with another embodiment of the present invention.

### DETAILED DESCRIPTION OF THE INVENTION

The detailed description and technical contents of the present invention will become apparent with the following detailed description accompanied with related drawings. It is noteworthy to point out that the drawings is provided for the illustration purpose only, but not intended for limiting the scope of the present invention.

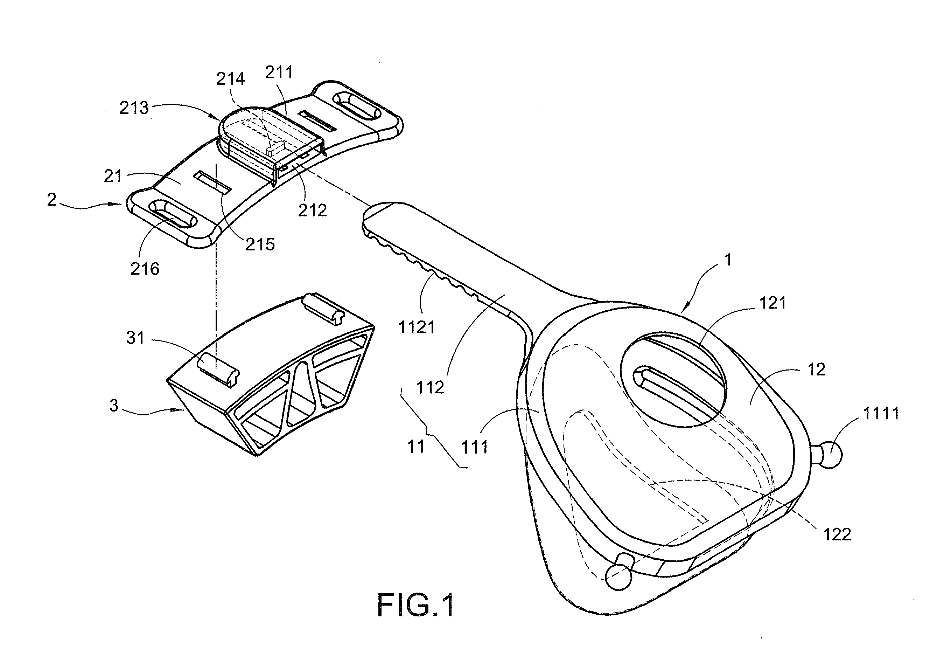

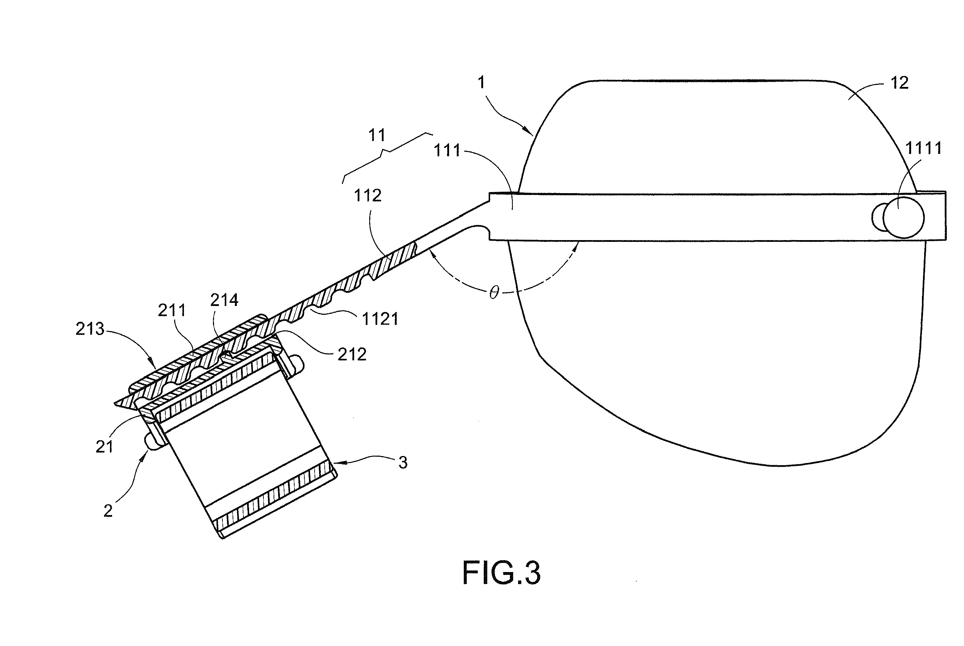

Please refer to FIGS. 1 to 3. The present invention provides an adjustable respiratory mask including a nasal cover 1 and a forehead fixing base 2.

The nasal cover 1 comprises a frame assembly 11 and a cover body 12. The frame assembly 11 has an upright frame 111 and an adjustable support 112. The upright frame 111 is provided on the periphery of the cover body 12. The adjustable support 112 is inclined relative to the upright frame 111. An angle (θ) is formed between the upright frame 111 and the adjustable support 112. The adjustable support 112 is a plastic piece, so that the adjustable support 112 can swing relative to the upright frame 111 and thus the angle (θ) is in a range of 90 to 270 degrees. The cover body 12 is made of silicon or rubber. The cover body 12 is provided with a first opening 121 and a second opening 122 opposite to the first opening 121. The upright frame 111 has a plurality of fixing portions 1111. The adjustable support 112 is provided with a plurality of engaging troughs 1121.

The forehead fixing base 2 has a base body 21. The base body 21 is provided with a protrusion 211. The protrusion 211 is provided with a through-hole 212 to form a receiving portion 213. The base body 21 is formed with an engaging piece 214 in the through-hole 212. The engaging piece 214 can be engaged with any one of the engaging troughs 1121, so that the adjustable support 112 penetrates the receiving portion 213 to allow the base body 21 to adjustably move. The base body 21 is provided with a plurality of through troughs 215 and a plurality of accommodating portions 216. Of course, conversely, the base body 21 may be also provided with a plurality of engaging troughs. The adjustable support 112 has an engaging piece engaged with any one of the engaging troughs.

The adjustable respiratory mask of the present invention further includes a protective pad 3 made of silicon or rubber, which will be described more detail as follows. The protective pad 3 has a plurality of engaging blocks 31. Each of the engaging blocks 31 penetrates the through trough 215 to be engaged therein. In this way, the protective pad 3 is fixed below the base body 21.

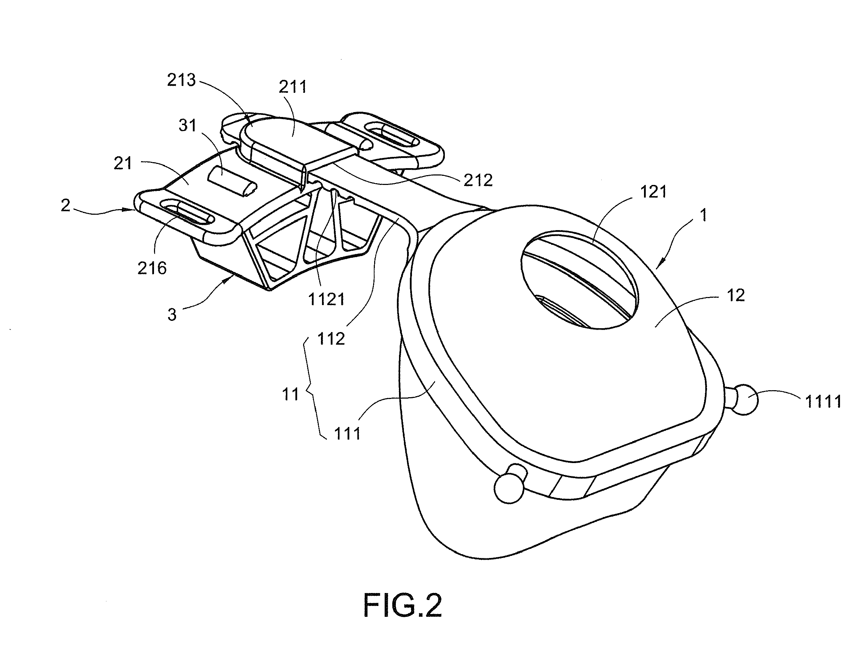

The assembly of the adjustable respiratory mask of the present invention will be described as follows with reference to FIGS. 2 to 3. The nasal cover 1 comprises the frame assembly 11 and the cover body 12. The frame assembly 11 has the upright frame 111 and an adjustable support 112 extending from the upright frame 111. The upright frame 111 is connected to the cover body 12. The adjustable support 112 is inclined relative to the upright frame 111. The forehead fixing base 2 has a base body 21 provided with a receiving portion 213. The adjustable support 112 penetrates the receiving portion 213 to allow the base body 21 to adjustably move. In this way, the adjustable support 112 penetrates and moves in the receiving portion 213, thereby adjusting the distance between the nasal cover 1 and the forehead fixing base 2. The adjustable support 112 is inclined relative to the upright frame 111, thereby adjusting the angle (θ) between the nasal cover 1 and the forehead fixing base 2. In this way, the face contours of different users can be fitted, and the user will feel more stable and comfortable when wearing it.

The adjustable support 112 is a plastic piece, so that it can swing relative to the upright frame 111 when in use. In this way, the angle (θ) between the nasal cover 1 and the forehead fixing base 2 can be adjusted, and the face contour of the user can be fitted. However, since the plastic piece may suffer from an elastic fatigue after being used for a period of time, it can memorize the special face contour of the user. Thus, the user can feel more stable and comfortable when wearing it.

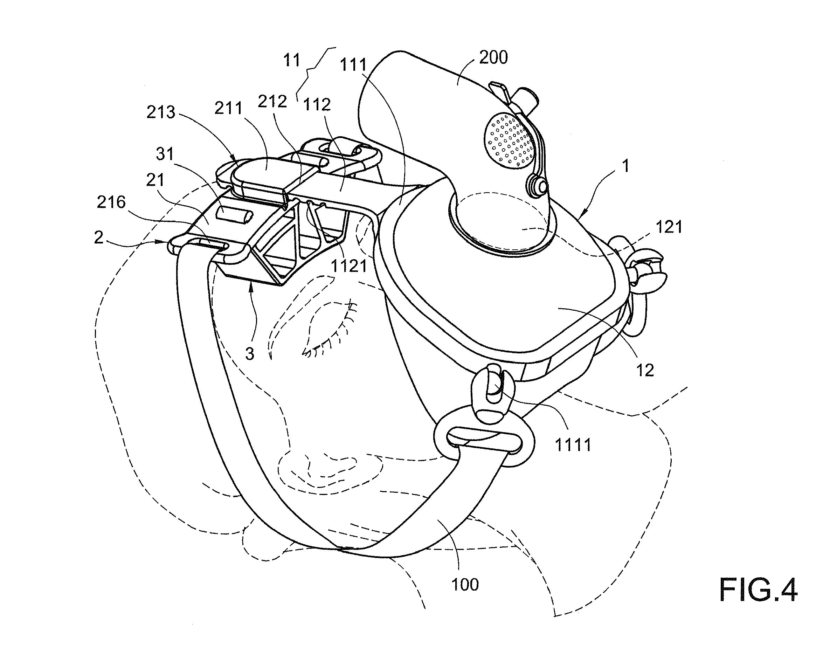

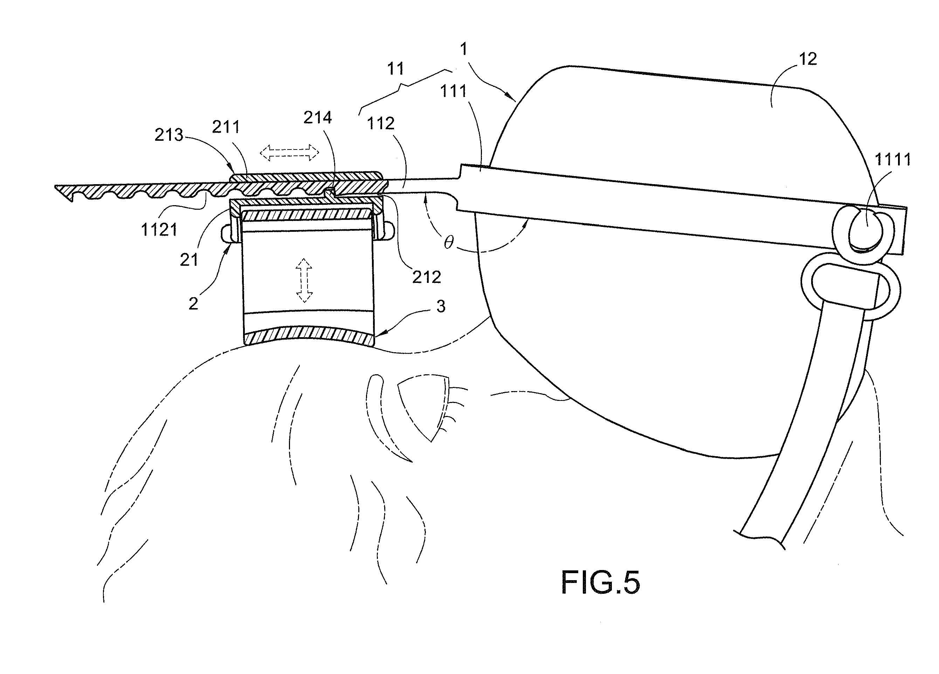

Please refer to FIGS. 4 and 5, which are schematic views showing the operating state of the respiratory mask of the present invention. The upright frame 111 is connected to the fixing band 100 via a fixing portion 1111 and the base body 21 is connected to the fixing band 100 via a accommodating portion 216. In this way, the respiratory mask can be put on the face of the user. The first opening 121 of the cover body 12 is connected to an air supply device 200, while the second opening 122 is disposed to cover the nose and mouth of the user. The engaging piece 214 is engaged with any one of the engaging troughs 1121, thereby adjusting the distance between the nasal cover 1 and the forehead fixing base 2 via the adjustable support 112, while adjusting the angle (θ) between the nasal cover 1 and the forehead fixing base 2. In this way, besides the adjustment in its front-and-rear direction, the forehead fixing base 2 can be slightly adjusted in height, so that the nasal cover 1 and the forehead fixing base 2 can more fit the face contour of the user. Thus, the cover body 12 can tightly cover the nose and mouth of the user, thereby avoiding the insufficient air pressure due to air leakage. Therefore, the respiratory mask of the present invention can be used more stable.

The respiratory mask of the present invention further includes a protective pad 3 made of silicon or rubber. The protective pad 3 can be plastically sandwiched between the forehead fixing base 2 and the forehead of the user. No matter the user wears the respiratory mask tight or loose, the protective pad 3 can provide a cushioning effect, so that the user can feel more comfortable when wearing the respiratory mask of the present invention.

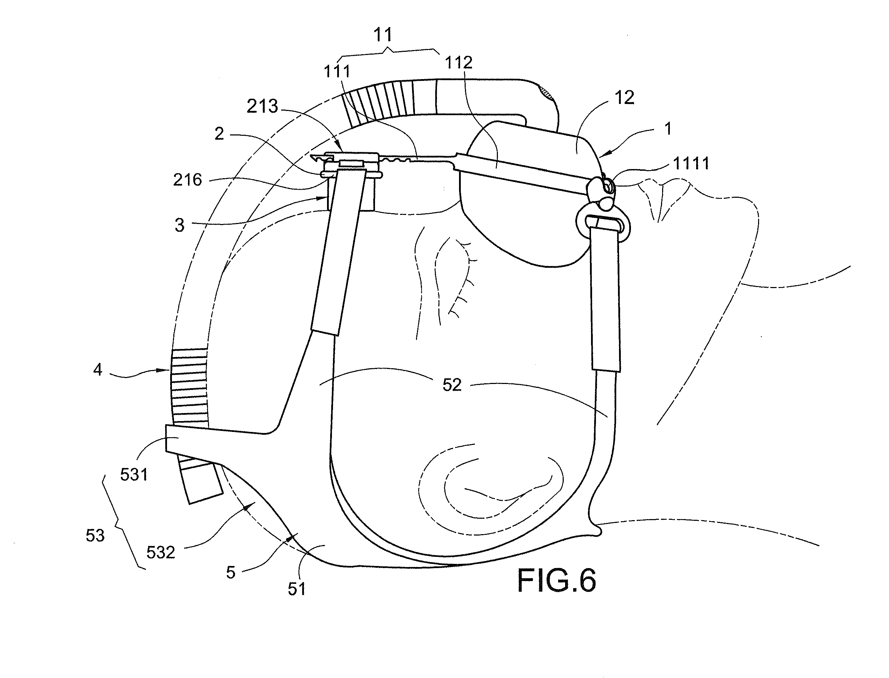

Please refer to FIG. 6, which is a schematic view showing the operating state of the respiratory mask in accordance with another embodiment of the present invention. The respiratory mask of the present invention further includes a connecting pipe 4 and a head band 5. The head band 5 has a main body 51. Both sides of the main body 51 extend to form at least one fixing band 52 respectively. The top of the main body 51 is formed with a penetrating portion 53. One end of the connecting pipe 4 is connected to the first opening 121, while the other end of the connecting pipe 4 is positioned to penetrate the penetrating portion 53. The penetrating portion 53 is an extension band 531 with its both ends connected to the main body 51. A hollow region 532 is formed between the extension band 531 and the main body 51. In this way, the fixing band 52 is connected to the accommodating portion 216 and the fixing portion 1111, so that the respiratory mask can be fixed onto the face of the user. The hollow region 532 is a space formed between the extension band 531 and the head of the user, so that the connecting pipe 4 can penetrate to be fixed into the hollow region 532. In this way, the connecting pipe 4 can be protected from swinging, so that the respiratory mask of the present invention can be used more conveniently.

In summary an adjustable respiratory mask is disclosed. The respiratory mask includes a nasal cover 1 and a forehead fixing base 2. The nasal cover 1 includes a frame assembly 11 and a cover body 12. The frame assembly 11 has an upright frame 111 and an adjustable support 112 extending from the upright frame 111. The upright frame 111 is connected to the cover body 12. The adjustable support 112 is a plastic piece and inclined relative to the upright frame 111. The forehead fixing base 2 has a base body 21 provided with a receiving portion 213. The adjustable support 112 penetrates the receiving portion 213 to allow the base body 21 to adjustably move. Thus, the respiratory mask of the present invention can fit face contours of different users, so that the users can feel more stable and comfortable when wearing it.

## Claims

1. An adjustable respiratory mask, including:
a nasal cover (1) comprising a frame assembly (11) and a cover body (12), the frame assembly (11) having an upright frame (111) and an adjustable support (112) extending from the upright frame (111), the upright frame (111) being connected to the cover body (12), the adjustable support (112) being a plastic piece and inclined relative to the upright frame (111); and
a forehead fixing base (2) having a base body (21), the base body (21) being provided with a receiving portion (213), the adjustable support (112) penetrating the receiving portion (213) to allow the base body (21) to adjustably move.

2. The adjustable respiratory mask according to claim 1, wherein an angle (θ) is formed between the upright frame (111) and the adjustable support (112), the angle (θ) is in a range between 90 to 270 degrees.

3. The adjustable respiratory mask according to claim 1 or 2, wherein the base body (21) has a protrusion (211), the protrusion (211) is provided with a through-hole (212) to form the receiving portion (213), the base body (21) is formed with an engaging piece (214) in the through-hole (212), the adjustable support (112) is provided with a plurality of engaging troughs (1121), the engaging piece (214) is engaged with any one of the engaging troughs (1121).

4. The adjustable respiratory mask according to one of the preceding claims, further including a protective pad (3) fixed below the base body (21).

5. The adjustable respiratory mask according to claim 4, wherein the protective pad (3) has a plurality of engaging blocks (31), the base body (21) is provided with a plurality of through troughs (215), the engaging blocks (31) penetrate the through troughs (215) to be engaged therein.

6. The adjustable respiratory mask according to claim 4, wherein the protective pad (3) is made of silicon or rubber.

7. The adjustable respiratory mask according to one of the preceding claims, wherein the cover body (12) is provided with a first opening (121) and a second opening (122) opposite to the first opening (121).

8. The adjustable respiratory mask according to one of the preceding claims" wherein the upright frame (111) has a plurality of fixing portions (1111).

9. The adjustable respiratory mask according to claim 1, wherein the base body (21) has a plurality of accommodating portions (216).

10. The adjustable respiratory mask according to one of the preceding claims,, further including a connecting pipe (4) and a head band (5), the head band (5) having a main body (51), both sides of the main body (51) extending to form at least one fixing band (52) respectively, the top of the main body (51) being formed with a penetrating portion (53), one end of the connecting pipe (4) penetrating the first opening (121), the other end of the connecting pipe (4) penetrating to be positioned into the penetrating portion (53).

11. The adjustable respiratory mask according to claim 10, wherein the penetrating portion (53) is an extension band (531) with its both ends connected to the main body (51), a hollow region (532) is formed between the extension band (531) and the main body (51).

## Amended claims

### Amended claims in accordance with Rule 137(2) EPC.

**1.** An adjustable respiratory mask, including:
a nasal cover (1) comprising a frame assembly (11) and a cover body (12), the frame assembly (11) having an upright frame (111) and an adjustable support (112) extending from the upright frame (111), the upright frame (111) being connected to the cover body (12), the adjustable support (112) being a plastic piece and inclined relative to the upright frame (111); and
a forehead fixing base (2) having a base body (21), the base body (21) being provided with a receiving portion (213), the adjustable support (112) penetrating the receiving portion (213);
wherein the upright frame (111) and the adjustable support (112) form an integral body;
wherein the base body (21) has a protrusion (211), the protrusion (211) is provided with a through-hole (212) to form the receiving portion (213), the base body (21) is formed with an engaging piece (214) in the through-hole (212), the adjustable support (112) is provided with a plurality of engaging troughs (1121), the engaging piece (214) is engaged with any one of the engaging troughs (1121);
the adjustable support (112) is configured to swing relative to the upright frame(111)..

**2.** The adjustable respiratory mask according to claim 1, wherein an angle (0) is formed between the upright frame (111) and the adjustable support (112), the angle (θ) is in a range between 90 to 270 degrees.

**3.** The adjustable respiratory mask according to one of the preceding claims, further including a protective pad (3) fixed below the base body (21).

**4.** The adjustable respiratory mask according to claim 3, wherein the protective pad (3) has a plurality of engaging blocks (31), the base body (21) is provided with a plurality of through troughs (215), the engaging blocks (31) penetrate the through troughs (215) to be engaged therein.

**5.** The adjustable respiratory mask according to claim 3, wherein the protective pad (3) is made of silicon or rubber.

**6.** The adjustable respiratory mask according to one of the preceding claims, wherein the cover body (12) is provided with a first opening (121) and a second opening (122) opposite to the first opening (121).

**7.** The adjustable respiratory mask according to one of the preceding claims,, wherein the upright frame (111) has a plurality of fixing portions (1111).

**8.** The adjustable respiratory mask according to claim 1, wherein the base body (21) has a plurality of accommodating portions (216).

**9.** The adjustable respiratory mask according to claim 6, further including a connecting pipe (4) and a head band (5), the head band (5) having a main body (51), both sides of the main body (51) extending to form at least one fixing band (52) respectively, the top of the main body (51) being formed with a penetrating portion (53), one end of the connecting pipe (4) is configured to penetrate the first opening (121), the other end of the connecting pipe (4) is configured to penetrate to be positioned into the penetrating portion (53).

**10.** The adjustable respiratory mask according to claim 9, wherein the penetrating portion (53) is an extension band (531) with its both ends connected to the main body (51), a hollow region (532) is formed between the head of the user, the extension band (531) and the main body (51) after the mounting of the mask and the connection pipe (4).
